# EUROPEAN PATENT APPLICATION

(11) **EP 3 034 102 A1**
(43) Date of publication of application: **22.06.2016**
(21) Application number: 14199122.4
(22) Date of filing: 19.12.2014
(51) Int. Cl.: A61L 27/12, A61L 27/38

(54) **Stem cell carrier and method for bone regeneration with 3D customized CAD/CAM using the carrier**

(71) Applicant: Ezekiel Co., Ltd., Taean-gun, Chungcheongnam-do 357-906 (KR)
(72) Inventor: Lee, Jae Joon, 357-906 Chungcheongnam-do (KR)
(74) Representative: Awapatent A/S

(57) **Abstract**

The present invention relates to a stem cell scaffold and provides a cubic structure formed of a porous biocompatible material and having a plurality of through-hole channels such that stem cell can be cultured under optimal conditions.

## Description

### BACKGROUND OF THE INVENTION

### 1. Field of the Invention

The present invention relates to a new technique for a stem cell carrier and, more particularly, to a stem cell carrier produced from a biocompatible material and a method for bone regeneration using 3D customized CAD/CAM, without using a graft that is simply filled in a defect in bone tissue.

### 2. Description of the Related Art

Stem cell is a primitive cell and is also referred to as a pluripotent cell or progenitor cell that can grow into any organs. Such stem cells include embryonic stem cells using human embryo and adult stem cells that continuously produce blood cells.

The present invention relates particularly to the delivery of adult stem cells, which are extracted from cord blood or adults' bone marrow, blood, etc. and refer to primitive cells just before differentiation into cells of specific organs such as bone, liver, blood, etc. Adult stem cells include hematopoietic stem cells, mesenchymal stem cells, and neural stem cells, etc. that have attracted much attention as materials for regenerative medicine. Besides, there are stem cells in liver, epidermis, pancreas, etc.

Cord blood (umbilical cord blood) contains large amounts of hematopoietic stem cells, and myeloid cells found in the bone marrow contain various types of stem cells including hematopoietic stem cells, which can produce blood and lymphocytes, mesenchymal stem cells, etc.

Adult stem cells are difficult to proliferate and have a strong tendency to differentiate easily. Instead, various types of adult stem cells can be used to regenerate organs required by the actual medicine and can differentiate to meet the characteristics of each organ after transplantation of stem cells.

Moreover, unlike embryonic stem cells extracted from human embryo, adult stem cells are extracted from body tissues that have already grown, such as bone marrow or brain cells, and thus can avoid the ethical controversy.

Technology related to fundamental and applied tissue engineering has been advanced for the purpose of developing transplantable artificial tissues as part of regenerative medicine. Specifically, studies including stem cell proliferation and differentiation, development of cytocompatible and biocompatible three-dimensional scaffolds, and construction of a variety of tissue engineering tools are now the most active research areas in regenerative medicine. Among them, three-dimensional scaffolds that are used to deliver stem cells or tissue cells therein are critical for the development of artificial tissues and organs.

Scaffold materials used for the regeneration of body tissues must act as a substrate or framework to which cells adhere to form three-dimensional tissues and must also function as a temporary barrier between transplanted cells and host cells, which mean that the materials should have non-toxic biocompatibility such that they do not cause blood clotting and inflammatory response after transplantation. In addition, the scaffold materials must be biodegradable in vivo at a desired time when the transplanted cells have grown sufficiently to the point of being able to adequately function as a tissue. Therefore, the scaffolds are produced from synthetic or natural polymers or composites thereof and are manufactured into three-dimensional structures which have a variety of morphologies and properties. Synthetic biodegradable polymers that are currently being widely used include polyglycolic acid (PGA), polylactic acid (PLA), polylactic acid-polyglycolic acid copolymer (PLGA), poly-ε-caprolactone (PCL), and derivatives and copolymers thereof. Naturally biodegradable polymers include collagen, alginate, hyaluronic acid, gelatin, chitosan, fibrin, etc. A variety of different forms of materials, such as sponges, gels, fibers, and microbeads, are applied for the fabrication of scaffolds, and the most popular ones are porous sponges and injectable hydrogels.

However, among various technical barriers in tissue engineering, the most critical one with regard to the scaffold is the development of a cytocompatible surface environment within the scaffold. In light of the fact that scaffolds are fundamental in providing a three-dimensional environment that is advantageous for cell adhesion and growth, the properties of the scaffold surface where cell adhesion occurs may play a critical role in determining the present and future behaviors of cells.

The polymer surface to which cells are directly attached is one of the most important factors in scaffold design. In general, cell adhesion to natural polymers is much easier to accomplish than that to synthetic polymers, and thus a method of surface coating or grafting of scaffolds with materials which cell surface receptors can recognize, such as fibronectin, an arginine-glycine-aspartic acid (RGD) peptide, vitronectin, laminin, etc., a method of treating a polymeric scaffold surface with a natural polymer, such as collagen, gelatin, fibrin, etc., or a method of preparing a hybrid scaffold from a mixture of synthetic and natural polymers has been conventionally used. However, these methods are only partly effective in ensuring cell adhesion and stem cell differentiation, but in fact, there are limitations in creating a biomimetic surface environment that the cells recognize as their own environment.

Meanwhile, extracellular matrix (ECM) is a matrix that surrounds cells, occupies space between cells, and has a network structure composed mainly of proteins and polysaccharides. Components of the extracellular matrix include structural elements such as collagen and elastin; adhesive proteins such as fibronectin, laminin, vitronectin, and tenascin; proteoglycans such as chondroitin sulfates or heparan sulfates and core proteins; and hyaluronic acids consisting of polysaccharides only. These extracellular matrix components are the major substances that determine the shapes of tissues formed by aggregating cells, provide an environment where cells have normal functions, and are involved in cell differentiation.

Therefore, many studies have been carried out, using the advantages of the extracellular matrix that provides an environment where cells have normal functions. For example, living tissues such as porcine small intestine submucosa (SIS), bladder, and skin, are directly decellularized to obtain a cell-free matrix, which is then used for cell transplantation. In addition, Korean Patent No. 10-715505 describes a method of preparing a cell-derived extracellular matrix scaffold. The cell-derived extracellular matrix scaffold is a porous scaffold produced from cartilage prepared and cultured by tissue engineering using a scaffold-free system configured to obtain a chondrocyte/extracellular matrix membrane from animal cartilage-derived chondrocytes, culture the obtained chondrocyte/extracellular matrix membrane into a scaffold-free pellet-type structure, and freeze-dry the pellet-type structure, and the prepared porous scaffold is useful only to cartilage regeneration.

Therefore, there is a need for developing a scaffold that provides a biomimetic environment that is useful even for regeneration of other tissue cells.

### SUMMARY OF THE INVENTION

An object of the present invention is to provide a stem cell carrier or scaffold which can deliver adult stem cells into the body and provide an environment where the delivered adult stem cells can differentiate into bone tissue.

Moreover, the stem cell carrier transplanted into the body can withstand the weight and other mechanical load in a solid state and allows stem cells to be delivered into the body during transplantation. The stem cell carrier can be fixed and maintained in a transplanted area without undesired deformation or displacement, and after the transplantation of the carrier without affecting the adjacent anatomical and surgical structure, it does not require an additional graft or device to more firmly engage the surface of the carrier with the surface of adjacent tissue.

Furthermore, the present invention provides a 3D bone scaffold that perfectly coincides with a bone defect using CAD/CAM, facilitating bone regeneration without any biological side effects.

In an embodiment of the present invention, the stem cell carrier is formed with a cubic structure having a plurality of through holes. That is, this cubic structure has a plurality of through-hole channels having a diameter of 400 to 500 micrometers formed with hydroxyapatite (HA) or hybrid materials thereof and thus provides a surface and a scaffold where stem cells can be cultured.

Moreover, in order to form a stem cell scaffold for bone regeneration, the bone defect is diagnosed and imaged using CT and MRI, and the image information is sent to a CAD/CAM to form the scaffold in a three-dimensional manner using the cubic structure.

Adult stem cells are stored in the formed scaffold of the carrier, which is then fixed to the bone defect.

### BRIEF DESCRIPTION OF THE DRAWINGS

The above and other features and advantages of the present invention will become more apparent by describing in detail exemplary embodiments thereof with reference to the attached drawings in which:
FIG. 1 is a perspective view showing a stem cell carrier with a cubic structure;
FIG. 2 is a plan view of FIG. 1; and
FIG. 3 is an enlarged perspective view of a single channel forming a part of FIG. 1.

### DETAILED DESCRIPTION OF THE INVENTION

Hereinafter, the present invention will be described in detail with reference to the accompanying drawings.

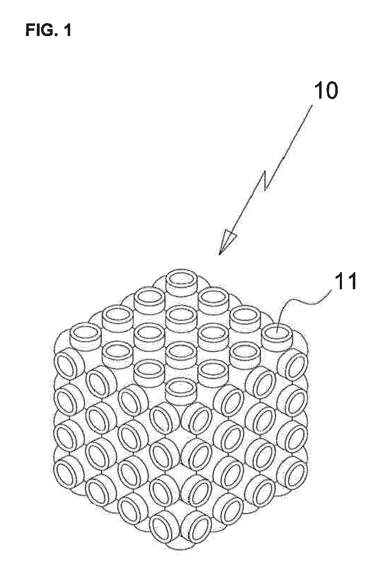

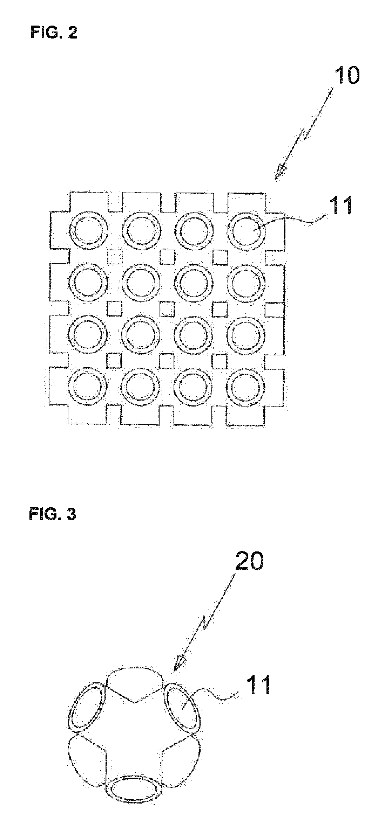

As shown in FIGS. 1 and 2, a stem cell carrier 10 has a cubic structure comprising a plurality of through holes 11 formed to penetrate through the cubic structure.

The stem cell carrier 10 is composed of a single body which may be divided by physical force into single units 20 as shown in FIG. 3.

The stem cell carrier 10 is composed of hydroxyapatite (HA), β-TCP (tricalcium phosphate), or mixtures thereof, and these materials are the main components that constitute bones and teeth of the body and have the advantages that they are more flexible than simple ceramic, have good biocompatibility, and do not cause deterioration and unnecessary biological reactions.

The roles of the stem cell carrier 10 are to activate cells in the body to secrete a new matrix, thus regenerating differentiated tissue. Stem cells are supported on a scaffold that is non-toxic to the body and is completely biodegradable, and the hydroxyapatite (HA) and β-TCP have sufficient effect as tissue engineering porous scaffolds.

A method for bone regeneration using the stem cell carrier 10 configured in the above-described manner will be described below.

First, a bond defect is imaged using CT and MRI, and the image information is sent to a CAD/CAM to form a scaffold using the stem cell carrier 10 prepared to coincide with the bone defect.

Stem cells are stored in the formed scaffold, and the scaffold is put on the bone defect and then fixed using a fixing means composed of a biocompatible material such as PLA.

As described above, according to the stem cell carrier of the present invention, the treatment of bone damage can reduce the risks of immune rejection and infection in patients and allows stem cells isolated from the body to be cultured and differentiated, thus maintaining homeostasis in the defect tissue.

## Claims

1. A stem cell carrier composed of a plurality of single units 20 made of a porous material to form a cubic structure, wherein a plurality of channels, each forming a through hole of 400 to 500 micrometers in diameter, are formed from one side of the cubic structure to the other side.

2. The stem cell carrier of claim 1, wherein the porous material is selected from the group consisting of hydroxyapatite (HA), β-TCP (tricalcium phosphate), and mixtures thereof.

3. A method for bone regeneration with 3D customized CAD/CAM, the method comprising the steps of:
imaging a bone defect using CT and MRI;
sending the image information to a CAD/CAM to form a scaffold using a stem cell carrier;
storing stem cells in the formed scaffold; and
fixing the scaffold storing the stem cells with a fixing means composed of a biocompatible material.
